# EUROPÄISCHE PATENTSCHRIFT

(11) **EP 3 317 438 B1**
(45) Veröffentlichungstag und Bekanntmachung des Hinweises auf die Patenterteilung: **31.07.2024**
(21) Anmeldenummer: 16733559.5
(22) Anmeldetag: 30.06.2016
(51) Int. Cl.: C25D 13/06, C09D 5/44, A61B 18/14

(54) **BESCHICHTUNG FÜR APPLIKATOREN IN DER ELEKTROCHIRURGIE**
COATING FOR APPLICATORS IN ELECTROSURGERY
REVÊTEMENT POUR DES APPLICATEURS DANS L'ÉLECTROCHIRURGIE

(30) Priorität: 02.07.2015 DE 102015212389
(43) Veröffentlichungstag der Anmeldung: 09.05.2018
(73) Patentinhaber: Aesculap AG, 78532 Tuttlingen (DE)
(72) Erfinder: ROTHWEILER, Christoph, 78166 Donaueschingen (DE); REINECKE, Holger, 79312 Emmingen (DE); WEISSHAUPT, Dieter, 78194 Immendingen (DE)
(74) Vertreter: Patentanwälte Ruff, Wilhelm, Beier, Dauster & Partner mbB
(86) Internationale Anmeldenummer: PCT/EP2016/065272
(87) Internationale Veröffentlichungsnummer: WO 2017/001548

(56) Entgegenhaltungen:
- EP-A1- 0 546 494
- EP-A1- 1 302 567
- US-A- 5 122 591
- US-A1- 2008 077 131

## Beschreibung

Die Erfindung betrifft zum einen ein Verfahren zum Aufbringen mindestens einer Beschichtung aus mindestens einem elektrisch isolierenden Kunststoff auf einen Applikator für Ströme, insbesondere HF-Ströme in der Chirurgie sowie einen derart beschichteten Applikator. Zum anderen betrifft die Offenbarung eine Suspension für die elektrophoretische Abscheidung mindestens einer Beschichtung auf einem Applikator für Ströme, insbesondere HF-Ströme in der Chirurgie.

Bei der sogenannten Elektrochirurgie (Hochfrequenz-Chirurgie, HF-Chirurgie) wird Wechselstrom mit hoher Frequenz durch den menschlichen Körper geleitet. Dabei wird die elektrische Energie des Wechselstroms im Körpergewebe in erster Linie in Wärmeenergie umgewandelt. Hauptsächlich in Abhängigkeit von der Form der sogenannten Aktivelektrode, die das chirurgische Instrument bildet, kann das Gewebe bei hohen Stromdichten durch die Elektrode geschnitten werden (Elektrotomie) oder bei geringeren Stromdichten zur Blutstillung koaguliert werden.

Bei der sogenannten monopolaren Technik der HF-Chirurgie wird ein Pol der Hochfrequenz-Spannungsquelle über eine möglichst große Fläche mit dem Patienten über eine Neutralelektrode verbunden. Der andere Pol der Spannungsquelle ist mit der Aktivelektrode, die das eigentliche chirurgische Instrument bildet, verbunden.

Bei der sogenannten bipolaren Technik der HF-Chirurgie werden zwei gegeneinander isolierte Elektroden, zwischen denen die HF-Spannung anliegt, direkt an die Operationsstelle geführt. Der Stromkreis wird über das zwischen den Elektroden liegende Gewebe geschlossen, so dass der Strom im Gegensatz zur monopolaren Technik nur durch einen kleinen Teil des Körpers fließt. Der durch den Stromfluss hervorgerufene thermische Effekt tritt dementsprechend in erster Linie nur im Gewebe zwischen den Elektroden auf.

Bekannte monopolare oder bipolare Instrumente zur Applikation von hochfrequenten Strömen zum Schneiden und/oder Koagulieren von Gewebe in der Chirurgie sind beispielsweise Pinzetten, Klemmen, Scheren oder Haken.

Bei vielen Anwendungsfällen der HF-Chirurgie, insbesondere bei der bipolaren Anwendungstechnik, ist es entweder zwingend erforderlich oder zumindest von Vorteil, die entsprechenden Instrumente oder Instrumententeile, die in der Regel aus metallischen und damit elektrisch leitfähigen Materialien gefertigt sind, mit einer (elektrischen) Isolierung zu versehen. Dies geschieht in der Regel mit Hilfe geeigneter, elektrisch isolierender Kunststoffe, die häufig als Beschichtungen auf die entsprechenden Instrumente oder Instrumententeile aufgebracht werden.

Eine ähnliche Thematik existiert u.a. auch bei der Stimulation von Nerven durch Strom mithilfe einer sogenannten Nervenstimulationsnadel. Hierbei kann beispielsweise ein Nerv gezielt lokalisiert und anschließend ggf. sogar betäubt werden (Regionalanästhesie).

Das Aufbringen entsprechender isolierender Beschichtungen ist nach derzeitigem Stand der Technik in vielen Fällen aufwendig oder nur schwer mit reproduzierbaren Ergebnissen durchführbar. So werden elektrisch isolierende Kunststoffe beispielsweise elektrostatisch oder durch Wirbelsinterverfahren abgeschieden. Bei solchen Verfahren lassen sich aber häufig keine exakt wiederholbaren Beschichtungsbedingungen realisieren. Auch das Einbringen unerwünschter Fremdkörper in die Beschichtung kann ein Problem darstellen. Schließlich lassen sich mit solchen Verfahren häufig keine Schichtdicken bereitstellen, die gezielt für den jeweiligen elektrisch isolierenden Kunststoff bestimmte Werte für die sogenannte (elektrische) Durchschlagsfestigkeit aufweisen. Dies führt dazu, dass derzeit in der Regel aus Sicherheitsgründen viel dickere isolierende Kunststoffschichten abgeschieden werden, als dies nach den Produktspezifikationen des jeweiligen Kunststoffs eigentlich notwendig wäre.

Die EP 0 546 494 A1 offenbart kathodisch abscheidbare Elektrotauchlackbäder, die bestimmte Additive zur Verbesserung der Oberflächen der abgeschiedenen Schichten enthalten. Diese Elektrotauchlackbäder sind ausschließlich zur Lackierung von Automobilkarossen vorgesehen. Die Thematik, elektrisch isolierende Kunststoffe auf Applikatoren für Ströme, insbesondere HF-Ströme in der Chirurgie, aufzubringen, ist in dieser Druckschrift nicht angesprochen. Dementsprechend spielt auch die Fragestellung, dass bestimmte Werte für die elektrische Durchschlagsfestigkeit erhalten werden sollen, in dieser Schrift keine Rolle.

In der EP 1 302 567 A1 geht es um die Beschichtung von Oberflächen aus Leichtmetalllegierungen, wie sie beispielsweise im Flugzeugbau, bei Kraftfahrzeugteilen und bei Gehäuseteilen elektronischer Geräte vorliegen. Das in dieser Schrift beschriebene Beschichtungsverfahren zeichnet sich dadurch aus, dass nicht nur eine elektrophoretische Lackierung der Oberflächen erfolgt, sondern zusätzlich eine (weitere) galvanische Beschichtung der elektrophoretisch lackierten Oberfläche. Auch hier wird die Thematik der Verwendung eines elektrisch isolierenden Kunststoffs zum Erhalt bestimmter Werte für die elektrische Durchschlagsfestigkeit bei geringen Schichtdicken nicht angesprochen. US 2008/077131 A1 offenbart ein Verfahren und einen Stromapplikator nach der Präambel von den Ansprüchen 1 und 12. US 5 122 591 A offenbart eine , für eine elektrophoretische Abscheidung geeignete Kunststoffsuspension mit einer Konzentration von 100 g/l und von 120 g/l.

Dementsprechend stellt sich die Erfindung die Aufgabe, insbesondere für die Beschichtung von Applikatoren der HF-Chirurgie mit elektrisch isolierenden Kunststoffen ein alternatives Beschichtungsverfahren zur Verfügung zu stellen. Insbesondere soll es mit Hilfe dieses alternativen Beschichtungsverfahrens möglich sein, die Dicke der jeweiligen Kunststoffbeschichtung bzw. Kunststoffbeschichtungen gezielt so einzustellen, dass bestimmte Werte für die elektrische Durchschlagsfestigkeit bei möglichst geringen (optimierten) Schichtdicken erreicht werden.

Diese Aufgabe wird gelöst durch das erfindungsgemäße Verfahren mit den Merkmalen des Anspruchs 1. Bevorzugte Ausführungen dieses Verfahrens sind in den Ansprüchen, die von diesem Verfahrensanspruch abhängig sind, definiert. Weiter ist Teil der Erfindung der erfindungsgemäße Applikator für Ströme, insbesondere HF-Ströme in der Chirurgie gemäß Anspruch 12. Bevorzugte Ausführungen des erfindungsgemäßen Applikators sind in den jeweils abhängigen Ansprüchen definiert.

Der Wortlaut sämtlicher Ansprüche wird hiermit durch Bezugnahme zum Inhalt dieser Beschreibung gemacht.

Erfindungsgemäß ist das Verfahren der eingangs genannten Art dadurch gekennzeichnet, dass die Beschichtung des elektrisch isolierenden Kunststoffs mindestens teilweise, vorzugsweise aber vollständig, durch elektrophoretische Abscheidung erzeugt wird. Diese elektrophoretische Abscheidung erfolgt aus einer Suspension des Kunststoffs in mindestens einem organischen Lösungsmittel. Insbesondere kann die Beschichtung dabei auch durch mindestens zwei, vorzugsweise mehrere Abscheidevorgänge/Abscheideschritte bereitgestellt werden.

Erfindungsgemäß weist die durch das Verfahren erhaltene Kunststoffbeschichtung eine elektrische Durchschlagsfestigkeit von mindestens 500 V/mm und eine Dicke zwischen 5 µm und 500 µm auf.

Die elektrophoretische Abscheidung (EPD: Electrophoretic Deposition) ist ein aus dem Stand der Technik bekanntes Verfahren, bei dem (elektrisch) geladene Teilchen in einem elektrischen Feld wandern und auf einer Elektrode abgeschieden werden. Je nach Ausgestaltung dieses Verfahrens können dabei Schichten/Beschichtungen auf einem Grundkörper, der in der Regel als Elektrode dient, abgeschieden werden oder Formkörper selbst ausgebildet werden.

Sofern - wie bei der vorliegenden Erfindung - ein Feststoff (hier der elektrisch isolierende Kunststoff) elektrophoretisch abgeschieden werden soll, ist es erforderlich, die Kunststoffpartikel in einem flüssigen Medium in Suspension zu bringen und mit einer elektrischen Ladung zu versehen. Bei einer Suspension handelt es sich bekanntlich um ein heterogenes Stoffgemisch aus einer Flüssigkeit (dem "Lösungsmittel") und darin fein verteilten Feststoffen. Zur Bereitstellung der Suspension werden die Feststoffpartikel, die in der Regel zunächst als Pulver oder Granulat vorliegen, im Lösungsmittel aufgeschlämmt, beispielsweise unter Rühren. Häufig werden zusätzlich Dispergiermittel, wie beispielsweise Tenside, eingesetzt, die die Feststoffpartikel in der Suspension in der Schwebe halten und deren Sedimentation behindern.

Im vorliegenden Fall erfolgt die elektrophoretische Abscheidung aus einer Suspension des elektrisch isolierenden Kunststoffs in mindestens einem organischen Lösungsmittel.

Das erfindungsgemäße Verfahren ist in der Lage, durch die elektrophoretische Abscheidung einen elektrisch isolierenden Kunststoff zuverlässig auf einen Applikator für HF-Ströme in der Chirurgie abzuscheiden. Dies wird im Folgenden noch detailliert erläutert. Entscheidend ist dabei das Erreichen einer ausreichenden Durchschlagsfestigkeit (angegeben in kV/mm). Die Durchschlagsfestigkeit ist diejenige elektrische Feldstärke, welche in einem Material (hier der Kunststoffbeschichtung) höchstens herrschen darf, ohne dass es zu einem Spannungsdurchschlag (Lichtbogen) kommt. Die Durchschlagsfestigkeit eines isolierenden Werkstoffs entspricht einer elektrischen Feldstärke, so dass sie dementsprechend auch als Durchschlagsfeldstärke bezeichnet werden kann.

Dabei ist im Zusammenhang mit der vorliegenden Erfindung wichtig, dass die Durchschlagsfestigkeit bei vielen isolierenden Stoffen, auch elektrisch isolierenden Kunststoffen, nicht proportional zur Dicke ist. Dieser Effekt resultiert darin, dass ggf. dünne Schichten sogar höhere Durchschlagsfestigkeiten besitzen als dicke Schichten. Auch diese Tatsache wird im Folgenden im Zusammenhang mit der Erfindung noch näher erläutert.

Bei einer ersten Gruppe von bevorzugten Ausführungsformen des erfindungsgemäßen Verfahrens handelt es sich bei dem aufgebrachten Kunststoff um einen transparenten Kunststoff. Dementsprechend hebt sich ein solcher Kunststoff optisch nicht von dem entsprechenden Applikator, d.h. insbesondere der (bipolaren) Klemme oder (bipolaren) Pinzette ab. Als Folge davon ist der Applikator auch nach Aufbringen der Beschichtung mit all seinen Details vom Anwender zu erkennen.

Bei einer zweiten Gruppe von bevorzugten Ausführungsformen des erfindungsgemäßen Verfahrens handelt es sich bei dem verwendeten elektrisch isolierenden Kunststoff um einen gefärbten Kunststoff, wobei diese Einfärbung vorzugsweise durch den Zusatz von Farbpigmenten im Kunststoff bereit gestellt ist. Auf diese Weise ist die Beschichtung ohne Weiteres gegenüber dem Grundkörper des Applikators optisch zu erkennen, so dass beschichtete und nicht beschichtete Teile des Applikators für den Anwender voneinander unterscheidbar sind. Dementsprechend können solche gefärbten Kunststoffbeschichtungen eine Kennzeichnungsfunktion gegenüber dem Anwender erfüllen, so dass beispielsweise unterschiedliche Materialien des Applikators oder unterschiedliche Dimensionen, Materialstärken u.dgl. verschiedener Applikatoren mit unterschiedlichen Farben gekennzeichnet sein können.

Grundsätzlich können nach der Erfindung die unterschiedlichsten Kunststoffe als Beschichtung eingesetzt werden, solange sie die erforderlichen Eigenschaften einer elektrischen Isolierung erfüllen. Bevorzugt sind solche Kunststoffe, die in einfacher Weise in Form von Partikeln, d.h. in der Regel in der Form eines Pulvers oder eines Granulats, bereit gestellt werden können, so dass mit geeigneten Lösungsmitteln Suspensionen für die elektrophoretische Abscheidung herstellbar sind.

Weiter bevorzugt sind dabei in besonderer Weise thermoplastische Kunststoffe, d.h. Kunststoffe, die innerhalb bestimmter Temperaturbereiche (thermoplastisch) reversibel verformbar sind. Ein solches einfaches Aufschmelzen des elektrisch isolierenden Kunststoffs ist ggf. von Vorteil, um die elektrophoretisch abgeschiedenen Beschichtungen durch eine nachgeschaltete Wärmebehandlung weiter in ihren Oberflächeneigenschaften zu optimieren. Auch dies wird im Folgenden noch näher erläutert.

Vorzugsweise kann es sich bei den erfindungsgemäß eingesetzten Kunststoffen um Polyamide (PA) handeln, deren Strukturen und Eigenschaften dem Fachmann ohne weiteres bekannt sind. Polyamide sind überwiegend teilkristalline Thermoplaste mit geringer elektrischer Leitfähigkeit.

Eine andere Gruppe bevorzugt verwendbarer Kunststoffe sind die Polyaryletherketone (PAEK), die in der Polymerkette Ether- und Keton-Gruppen enthalten. Auch diese Kunststoffe sind dem Fachmann ohne weiteres bekannt, wobei ein besonders bevorzugtes und bekanntes Mitglied dieser Kunststoffgruppe Polyetheretherketon (PEEK) ist.

In besonderer Weise bevorzugt für den Einsatz im erfindungsgemäßen Verfahren sind die sogenannten Fluorkunststoffe als teilkristalline Thermoplaste aus perfluorierten Monomeren. In diesen Kunststoffen sind die Wasserstoffatome der Kohlenstoffhauptketten ganz oder teilweise durch Fluoratome ersetzt. Zu den Fluorkunststoffen gehört beispielsweise das (allerdings nicht schmelzbare) Polytetrafluorethylen (PTFE). Zu den Fluorkunststoffen gehören auch die hier bevorzugten thermoplastischen Fluorkunststoffe, von denen u.a. das Polyvinylidenfluorid (PVDF) zu nennen ist.

Besonders bevorzugt unter den thermoplastischen Fluorkunststoffen ist Polychlortrifluorethylen (PCTFE) und Ethylenchlortrifluorethylen (ECTFE). Bei beiden Kunststoffen handelt es sich um teilkristalline, thermoplastische Polymere mit guter Festigkeit und Härte, die als Pulver oder Granulate in Suspension für die elektrophoretische Abscheidung überführt werden können.

Ein bekanntes Produkt von ECTFE ist Halar^{®} ECTFE von Solvay Solexis, ein 1:1 alternierendes Copolymer von Ethylen und Chlortrifluorethylen.

In Weiterbildung ist es bei dem erfindungsgemäßen Verfahren bevorzugt, wenn vor dem Aufbringen der Kunststoffbeschichtung eine sogenannte Primerschicht aufgebracht wird. Derartige Primerschichten, die auch als Haftvermittlerschichten oder Grundierungsschichten bezeichnet werden können, dienen zur Haftverbesserung der Kunststoffschicht auf der Oberfläche des Applikators. Dabei kann nur eine Primerschicht vorgesehen sein oder auch ein sogenanntes Primersystem, das aus mehreren Schichten bestehen kann. Das Aufbringen der Primerschicht kann dabei durch die unterschiedlichsten, im Stand der Technik bekannten Methoden erfolgen, insbesondere ebenfalls durch elektrophoretische Abscheidung.

Weiter kann insbesondere vor dem Aufbringen der Kunststoffbeschichtung mindestens eine farbgebende Schicht aufgebracht werden. Diese farbgebende Schicht kann ebenfalls durch elektrophoretische Abscheidung aufgebracht werden. Dabei kann dann eine Suspension eines Farbpigments in einem organischen Lösungsmittel, insbesondere einem Alkohol, z.B. Isopropanol, vorzugsweise mit einem Tensid wie Natriumdodecylsulfat (SDS) zum Einsatz kommen. Entsprechende Pigmente sind beispielsweise PTFE Trockenpigmente schwarz, PTFE Trockenpigmente blau und Trockenpigmente Nickeltitangelb, wie sie beispielsweise von der Firma Colorant Chromatics AB, Finnland bezogen werden können.

Die farbgebende Schicht kann dann entweder mit einer transparenten Kunststoffbeschichtung oder einer eingefärbten Kunststoffbeschichtung nach dem erfindungsgemäßen Verfahren beschichtet/überschichtet werden. Das Aufbringen der farbgebenden Schicht(en) kann dabei vorzugsweise ebenfalls mit Hilfe der elektrophoretischen Abscheidung erfolgen. Dies hat den Vorteil, dass sowohl farbgebende Schicht als auch Kunststoffschicht (und ggf. auch Primerschicht) mit derselben Apparatur auf den Applikator aufgebracht werden können.

In diesem Zusammenhang ist es bei bevorzugten Ausführungsformen auch möglich, Pigmente in eine Primerschicht oder ein Primersystem einzubringen, und auf diese Weise mit Hilfe dieser Primerschichten einen farbgebenden Effekt auf dem Applikator zu erzeugen.

Für die Herstellung der entsprechenden Suspensionen für die elektrophoretische Abscheidung können grundsätzlich Partikel, d.h. Kunststoffteilchen oder Pigmentteilchen unterschiedlicher Größe eingesetzt werden. Dabei ist die Verwendung von Partikeln mit kleiner Größe bevorzugt, da auf diese Weise dünnere Schichten erhalten werden können. Auch die im folgenden noch beschriebene Nachbehandlung der erhaltenen Schichten durch nachträgliches Aufschmelzen führt zu besseren Resultaten, wenn in der zur elektrophoretischen Abscheidung eingesetzten Suspension Partikel mit vergleichsweise geringen Durchmessern vorhanden sind. Auch an Kanten und Ecken der zu beschichteten Applikatoren, insbesondere an abgerundeten Ecken und Kanten lassen sich Beschichtungen mit weitgehend konstanter Schichtdicke besser realisieren, wenn in den Suspensionen Partikel mit vergleichsweise kleinen Durchmessern enthalten sind.

Ein geeignetes Maß für die Verteilung der Partikelgrößen eines eingesetzten Kunststoffmaterials oder eines eingesetzten Pigments ist der sogenannte D50-Wert, der für die sogenannte mittlere Partikelgröße steht. D50 bedeutet, dass 50 % der Partikel kleiner sind als der angegebene Wert.

Im Zuge dieser Definition ist es bei dem erfindungsgemäßen Verfahren bevorzugt, wenn der D50-Wert der eingesetzten Partikel (Kunststoff, Primer, Pigment) kleiner als 100 µm, vorzugsweise kleiner 80 µm, insbesondere kleiner 40 µm beträgt. In diesem Zusammenhang ist es möglich, ein kommerziell erhältliches Produkt mit größerer Partikelgröße vor Überführen in die entsprechende Suspension entsprechend zu bearbeiten, beispielsweise durch mindestens einen Mahlvorgang. Auch mithilfe der nachträglichen Bearbeitung einer bereits hergestellten Suspension mit Ultraschall können die in der Suspension enthaltenen Partikel (Kunststoff, Primer, Pigment) in ihrem Durchmesser reduziert werden, so dass auf diese Weise geringere D50-Werte der Partikel in der Suspension erreicht werden können.

Ein geeignetes Messverfahren zur Bestimmung der Partikelgröße ist beispielsweise die Laserdiffraktometrie, wie sie üblicherweise zur Bestimmung der Partikelgröße unter anderem in Suspensionen nach dem Stand der Technik eingesetzt wird.

Weiter ist es bei dem erfindungsgemäßen Verfahren bevorzugt, wenn die durch das Verfahren erhaltene Kunststoffbeschichtung eine elektrische Durchschlagsfestigkeit von mindestens 1200 V/mm, insbesondere mindestens 2500 V/mm aufweist.

Die nach dem erfindungsgemäßen Verfahren erhaltene Kunststoffbeschichtung besitzt bei der Erfindung vorzugsweise eine Dicke von 30 µm bis 350 µm. Innerhalb des zuletzt genannten Bereichs sind Schichtdicken von 100 µm bis 250 µm weiter bevorzugt.

Weiterhin sind bei der Erfindung Kunststoffbeschichtungen mit Schichtdicken zwischen 5 µm und 100 µm vorgesehen, wobei Schichtdicken zwischen 10 µm und 60 µm, insbesondere zwischen 15 µm und 30 µm weiter bevorzugt sind. Solche Schichtdicken sind in besonderer Weise bei der Beschichtung den eingangs genannten Nervenstimulationsnadeln vorgesehen.

Wie bereits erläutert, ist es bei der Erfindung von Vorteil, wenn bestimmte erwünschte Werte für die elektrische Durchschlagsfestigkeit bei bestimmten, insbesondere möglichst geringen, Schichtdicken der Kunststoffbeschichtung erreicht werden. Dementsprechend sind beim erfindungsgemäßen Verfahren Ausführungen bevorzugt, bei denen bei den oben genannten Schichtdicken der Kunststoffbeschichtung die elektrische Durchschlagsfestigkeit zwischen 3 kV/mm und 150 kV/mm, insbesondere zwischen 50 kV/mm und 100 kV/mm, beträgt.

Insbesondere bei solchen Ausführungen des erfindungsgemäßen Verfahrens ist gewährleistet, dass für jeden verwendeten elektrisch isolierenden Kunststoff bestimmte, beispielsweise die vom Hersteller angegebenen Werte für die elektrische Durchschlagsfestigkeit bei definierten Schichtdicken zur Verfügung gestellt werden. Dies wird erreicht durch die elektrophoretische Abscheidung der Kunststoffpartikel aus einer Suspension in einem organischen Lösungsmittel.

Die aufzubringende Schichtdicke wird dabei für jedes Kunststoffmaterial ausgehend von der maximalen Spannung, den die mit dem Applikator zu verwendende Spannungsquelle bereitstellt, bestimmt. Beispielsweise kann diese (maximale) Peakspannung Vₚ 600 Volt betragen.

Die elektrophoretische Abscheidung der Kunststoffbeschichtung kann bei der Erfindung grundsätzlich mit allen Spannungswerten erfolgen, die bei der elektrophoretischen Abscheidung üblich sind. Vorzugsweise wird die Kunststoffbeschichtung bei einer Spannung zwischen 0,2 kV und 4 kV, insbesondere zwischen 0,5 kV und 2 kV, erzeugt. Innerhalb des zuletzt genannten Bereichs sind Spannungswerte für die elektrophoretische Abscheidung zwischen 0,8 kV und 1,2 kV weiter bevorzugt.

Auch der Zeitraum, innerhalb dessen die elektrophoretische Abscheidung der Kunststoffbeschichtung erfolgt, ist erfindungsgemäß frei wählbar. Hier sind Zeiträume zwischen 5 Sekunden und 10 Minuten bevorzugt, wobei Zeiträume zwischen 5 Sekunden und 60 Sekunden, insbesondere zwischen 10 Sekunden und 40 Sekunden, weiter hervorzuheben sind.

In Weiterbildung ist es bei dem erfindungsgemäßen Verfahren bevorzugt, wenn die elektrophoretische Abscheidung bei einer Temperatur zwischen 10 °C und 80 °C, insbesondere zwischen 20 °C und 60 °C, durchgeführt wird. Dabei ist es grundsätzlich von Vorteil, wenn die Temperatur während der elektrophoretischen Abscheidung konstant gehalten wird, um eine bessere Reproduzierbarkeit der Abscheidung zu gewährleisten.

Grundsätzlich können bei der Erfindung verschiedene organische Lösungsmittel oder Lösungsmittelgemische zur Herstellung der entsprechenden Suspension der Kunststoffpartikel eingesetzt werden. Eine mit Vorteil verwendbare Gruppe von organischen Lösungsmitteln sind organische Alkohole, insbesondere Alkanole, wie Ethanol oder Isopropanol. Vorzugsweise handelt es sich bei den organischen Lösungsmitteln auch um aprotische, insbesondere aprotischunpolare Lösungsmittel, insbesondere um Alkane. Unter diesen Alkanen sind insbesondere die Hexane und Heptane, vorzugsweise n-Hexan und n-Heptan, hervorzuheben.

Wie bereits erwähnt, können der Suspension beim erfindungsgemäßen Verfahren Dispergiermittel, insbesondere Tenside, zugesetzt sein. Derartige Tenside sind dem Fachmann bekannt, wobei hier lediglich Natriumdodecylsulfat (SDS) als Beispiel genannt werden soll.

In Weiterbildung ist es bei dem erfindungsgemäßen Verfahren bevorzugt, wenn der mit der Kunststoffbeschichtung versehene Applikator nach der elektrophoretischen Abscheidung getrocknet wird. Dieses Trocknen dient in erster Linie zum Entfernen von überschüssigem organischem Lösungsmittel von der Oberfläche der Beschichtung. Dabei erfolgt ein solcher Trocknungsvorgang in der Regel deutlich unterhalb der Schmelztemperatur des entsprechenden elektrisch isolierenden Kunststoffs.

In Weiterbildung wird die durch elektrophoretische Abscheidung erzeugte Kunststoffbeschichtung bei dem erfindungsgemäßen Verfahren einer Nachbehandlung durch Aufschmelzen unterzogen. Diese Nachbehandlung dient dazu, die Qualität der Kunststoffbeschichtung, insbesondere der Oberfläche dieser Kunststoffbeschichtung, weiter zu verbessern, und kann bei der Erfindung vorzugsweise nach jedem elektrophoretischen Abscheideschritt durchgeführt werden. Durch das Aufschmelzen werden ggf. vorhandene Defekte, wie Poren, in der Beschichtung mindestens teilweise geschlossen und/oder ggf. vorhandene Unebenheiten der Beschichtung mindestens teilweise ausgeglichen. Dementsprechend kann man hier auch von einem Homogenisieren der Kunststoffbeschichtung durch Aufschmelzen sprechen. Auch Übergänge mit allmählich abnehmender Schichtdicke zwischen beschichteten und unbeschichteten Bereichen auf dem Applikator lassen sich mit Hilfe des Aufschmelzens gut realisieren. Diese Art der Nachbehandlung lässt sich in besonders vorteilhafter Weise bei thermoplastischen Kunststoffen, wie sie bei der Erfindung Verwendung finden können, durchführen.

Grundsätzlich ist die genannte Nachbehandlung bei beliebigen Temperaturen möglich, bei denen zumindest eine Erweichung des entsprechenden Kunststoffs eintritt. Dementsprechend bildet diese Erweichungstemperatur die Untergrenze einer sinnvollen Nachbehandlung. Die Temperaturobergrenze sollte maximal 20 % oberhalb der Schmelztemperatur des Kunststoffs liegen, damit die Struktur der Beschichtung insgesamt nicht gefährdet wird. Vorzugsweise erfolgt die Nachbehandlung erfindungsgemäß bei einer Temperatur von ca. 5 % bis 15 % oberhalb der Schmelztemperatur des Kunststoffs, insbesondere bei einer Temperatur von ca. 10 % oberhalb der Schmelztemperatur.

Bei den genannten Verfahren mit einer Nachbehandlung durch Aufschmelzen ist es grundsätzlich von Vorteil, wenn der beschichtete Applikator innerhalb eines möglichst kurzen Zeitraums auf die entsprechende Nachbehandlungstemperatur gebracht wird, um eine Rissbildung in der Kunststoffbeschichtung zu vermeiden.

Erfindungsgemäß kann die Nachbehandlung so lange erfolgen, bis sämtliche Defekte in der Kunststoffbeschichtung beseitigt und deren Oberfläche nivelliert ist. Bevorzugte Zeiträume für eine solche Nachbehandlung liegen unterhalb von 1 Stunde und betragen vorzugsweise weniger als 30 Minuten, insbesondere weniger als 15 Minuten.

Dementsprechend ist ein besonders bevorzugtes erfindungsgemäßes Verfahren gekennzeichnet durch die folgende Verfahrensabfolge:
- Zunächst wird der unbeschichtete Applikator, sofern erforderlich oder zweckmäßig, entfettet,
- ggf. wird der insbesondere entfettete Applikator mit mindestens einem Lösungsmittel, insbesondere einem organischen Lösungsmittel, gespült und anschließend getrocknet,
- ggf. wird auf den Applikator dann eine Primerschicht aufgebracht, vorzugsweise durch elektrophoretische Abscheidung,
- dann wird ggf. eine farbgebende Schicht aufgebracht, vorzugsweise ebenfalls durch elektrophoretische Abscheidung,
- in einem sich daran anschließenden Verfahrensschritt wird mindestens eine Schicht mindestens eines elektrisch isolierenden Kunststoffs auf den ggf. vorbeschichteten Applikator aufgebracht und zwar erfindungsgemäß durch elektrophoretische Abscheidung,
- zum Aufbringen mehrerer Schichten des elektrisch isolierenden Kunststoffs kann der zuvor genannte Verfahrensschritt mindestens einmal wiederholt werden, wobei ggf. die folgenden Verfahrensschritte nach jedem Abscheideschritt ebenfalls wiederholt werden können,
- ggf. wird die auf diese Weise erhaltene Kunststoffbeschichtung mit einem Lösungsmittel behandelt, insbesondere gespült,
- dann erfolgt ggf. eine thermische Behandlung des beschichteten Applikators zur Trocknung, vorzugsweise bei einer Temperatur deutlich unterhalb der Schmelztemperatur des elektrisch isolierenden Kunststoffs, und
- dann erfolgt ggf. eine thermische Nachbehandlung der erhaltenen Kunststoffbeschichtung durch Aufschmelzen dieser Beschichtung.

Weiter umfasst die Offenbarung eine Suspension für die elektrophoretische Abscheidung mindestens einer Beschichtung auf einem Applikator für Ströme, insbesondere HF-Ströme in der Chirurgie, insbesondere auf einer Klemme oder auf einer Pinzette oder auf einer Schere. Die Suspension umfasst mindestens einen elektrisch isolierenden Kunststoff in mindestens einem organischen Lösungsmittel.

Vorzugsweise beträgt die Konzentration des Kunststoffs in der Suspension zwischen 10 g/l und 150 g/l, insbesondere zwischen 20 g/l und 100 g/l. Die Viskosität der Suspension beträgt vorzugsweise zwischen 0,1 mPa·s und 10 mPa·s.

Bezüglich der in der Suspension enthaltenen Kunststoffe und organischen Lösungsmittel wird in vollem Umfang auf die entsprechenden Ausführungen im Zusammenhang mit dem erfindungsgemäßen Verfahren Bezug genommen und verwiesen. Insbesondere handelt es sich bei dem elektrisch isolierenden Kunststoff um ein Polyamid, um ein Polyaryletherketon, vorzugsweise um ein Polyetheretherketon (PEEK), oder um einen Fluorkunststoff, vorzugsweise einen thermoplastischen Fluorkunststoff, insbesondere um Polytetrafluorethylen (PTFE) oder insbesondere um Polychlortrifluorethylen (PCTFE) oder Ethylenchlortrifluorethylen (ECTFE).

Bei dem in der Suspension vorhandenen Lösungsmittel handelt es sich vorzugsweise um ein Alkan, insbesondere um n-Hexan und/oder n-Heptan.

Schließlich umfasst die Erfindung noch einen Applikator für Ströme, insbesondere HF-Ströme in der Chirurgie, insbesondere eine Klemme, Pinzette oder Schere, mit mindestens einer Beschichtung aus mindestens einem elektrisch isolierenden Kunststoff. Dieser Applikator ist erfindungsgemäß dadurch gekennzeichnet, dass der Kunststoff durch elektrophoretische Abscheidung aus einer Suspension des Kunststoffs in mindestens einem organischen Lösungsmittelaufgebracht ist und dass die erhaltene Kunststoffbeschichtung eine elektrische Durchschlagsfestigkeit von mindestens 500 V/mm und eine Dicke zwischen 5 µm und 500 µm aufweist.

Bei dem auf den Applikator aufgebrachten Kunststoff handelt es sich vorzugsweise um ein Polyamid, um ein Polyaryletherketon, vorzugsweise um ein Polyetheretherketon (PEEK), oder um einen Fluorkunststoff, vorzugsweise einen thermoplastischen Fluorkunststoff, insbesondere um Polytetrafluorethylen (PTFE) oder insbesondere um Polychlortrifluorethylen (PCTFE) oder Ethylenchlortrifluorethylen (ECTFE)

Wie bereits erläutert zeichnet sich der beschichtete Applikator gemäß der Erfindung dadurch aus, dass gezielt bestimmte Werte für die Durchschlagsfestigkeit bei bestimmten, insbesondere möglichst geringen Schichtdicken der Kunststoffbeschichtung bereitgestellt werden können. Das Aufbringen möglichst dicker Beschichtungen aus Sicherheitsgründen ist bei den erfindungsgemäß beschichteten Applikatoren nicht notwendig. Dementsprechend ist es bei der Erfindung bevorzugt, wenn die erfindungsgemäßen Applikatoren elektrische Durchschlagsfestigkeiten zwischen 3 kV/mm und 150 kV/mm, insbesondere zwischen 50 kV/mm und 100 kV/mm, bereitstellen, bei den oben genannten Dicken der Kunststoffbeschichtung, vorzugsweise zwischen 5 µm und 100 µm, insbesondere zwischen 10 µm und 60 µm.

Weitere Merkmale und Vorteile der Erfindung ergeben sich aus den nachfolgend beschriebenen Beispielen in Verbindung mit den Ansprüchen, ohne dass der Gegenstand der Erfindung auf die Beispiele zu beschränken ist. Hierbei können die offenbarten Merkmale jeweils für sich alleine oder in Kombination miteinander verwirklicht sein.

### Beispiele

Zur Durchführung der elektrophoretischen Abscheidung wurde ein Gefäß zur Aufnahme der Suspension des abzuscheidenden, elektrisch isolierenden Kunststoffs sowie eine Hochspannungsquelle, welche bis ca. 4 kV Ausgangsspannung liefert, bereitgestellt. Weiter wurde ein Rührer zum Rühren der Suspension während der elektrophoretischen Abscheidung vorgesehen. Schließlich war eine Gegenelektrode vorhanden, die während des Abscheidevorgangs als Anode geschaltet war.

Als zu beschichtende Applikatoren für die HF-Chirurgie wurden in den Beispielen Pinzetten für die bipolare Technik, sogenannte Bipolar-Pinzetten, verwendet. Diese Pinzetten waren während der elektrophoretischen Abscheidung als Kathode geschaltet.

Bei den Pinzetten waren an sämtlichen Oberflächenbereichen, die mit der Kunststoffbeschichtung versehen werden sollten, die Kanten abgerundet, um auf diese Weise eine bessere Beschichtungsqualität mit vorzugsweise gleichförmiger Schichtdicke zu erreichen. Außerdem waren die entsprechenden Oberflächen der Pinzetten mit Glasperlen abgestrahlt, um auf diese Weise hochfeine, reine Oberflächen für die Kunststoffbeschichtung bereitzustellen.

Als elektrisch isolierender Kunststoff wurde der thermoplastische Fluorkunststoff Halar^{®}ECTFE von Solvay Solexis eingesetzt und zwar das Produkt Halar^{®} 6014. Hierbei handelt es sich um ein transparentes Ethylenchlortrifluorethylen-Copolymer (ECTFE), bei dem die Bausteine Ethylen und Chlortrifluorethylen im Copolymer 1:1 alternieren. Die durchschnittliche Partikelgröße von Halar^{®} 6014 beträgt 80 µm (ASTM D 1921-63; Method C). Die Schmelztemperatur liegt bei 225 °C.

Weiter wurden unter Verwendung von Trockenpigmenten gefärbte Varianten des Halar^{®}-Kunststoffs hergestellt und zwar ein blaugefärbtes Halar, ein gelbgefärbtes Halar und ein schwarzgefärbtes Halar. Die Blau- und die Schwarzfärbung wurde durch PTFE-Pigmente erzielt, die Gelbfärbung durch ein Nickeltitangelb-Trockenpigment.

Weiter kam in den Beispielen ein Primer zum Einsatz, nämlich der Halar^{®}-Primer 6514, bei dem es sich ebenfalls um ein Ethylenchlortrifluorethylen-Copolymer handelt. Dieses Copolymer ist ein schwarzes Pulver mit einer durchschnittlichen Partikelgröße von 80 µm. Die Schmelztemperatur diese Copolymers beträgt ebenfalls 225 °C.

Aus den genannten Kunststoffen (transparentes ECTFE, gefärbtes ECTFE, ECTFE-Primer) wurden unter Verwendung des organischen Lösungsmittels n-Heptan Suspensionen für die elektrophoretische Abscheidung hergestellt. Dabei wurden sowohl Suspensionen bereitgestellt, die ein Dispergiermittel, nämlich Natriumdodecylsulfat (SDS) enthielten, als auch solche, die frei von Dispergiermitteln waren.

Bei sämtlichen Beispielen wurde dann wie folgt vorgegangen.

Zunächst wurden die zu beschichtenden Pinzetten entfettet, wobei eine anodische Heißentfettung eingesetzt wurde. Die Prozesstemperatur lag dabei zwischen 80 °C und 100 °C. Die Entfettung konnte dabei entweder im alkalischen Milieu (z.B. Natronlauge oder Kalilauge) oder im sauren Milieu (z.B. Phosphorsäure) stattfinden.

Dann wurden die so entfetteten Pinzetten mit einem geeigneten Medium gespült, beispielsweise mit Wasser (Reinstwasser) oder alternativ mit einem Alkohol, insbesondere Ethanol, oder mit einem Alkan, insbesondere n-Hexan. Dieser Spülprozess erfolgte bei Raumtemperatur.

Dann wurden die entsprechenden Kunststoffschichten (transparentes ECTFE, gefärbtes ECTFE und/oder ECTFE-Primer) durch elektrophoretische Abscheidung aufgebracht, wobei bei den entsprechenden Versuchen eine elektrophoretische Abscheidung mit einer Spannung von 2 kV über einen Zeitraum von 10 Sekunden durchgeführt wurde. Mit den entsprechenden Verfahrensparametern konnten nacheinander wahlweise mehrere Schichten des gleichen Materials oder Schichten unterschiedlicher Materialien übereinander auf die Applikatoroberfläche aufgebracht werden.

Die Konzentration der abzuscheidenden Kunststoffpartikel in der Suspension betrug 100 g/l.

Dann wurden die erhaltenen Schichten mit einem organischen Lösungsmittel, beispielsweise einem Alkohol, insbesondere Ethanol, oder einem Alkan, insbesondere n-Hexan, abgespült. Dieser Spülvorgang erfolgte in der Regel bei Raumtemperatur.

Dann wurden die erhaltenen Beschichtungen zunächst bei Temperaturen im Bereich zwischen 60 °C und 80 °C getrocknet und anschließend unter zumindest teilweisem Aufschmelzen des Kunststoffs thermisch nachbehandelt. Dies erfolgte bei einer Prozesstemperatur zwischen ca. 250 °C und 270 °C.

Bei der elektrophoretischen Abscheidung mehrerer Schichten übereinander wurden die Folgeschritte (Spülen, Trocknen, Aufschmelzen) nach jedem Abscheideschritt durchgeführt.

Gemäß den Beispielen wurden bei den angegebenen Verfahrensparametern bei einmaliger Abscheidung der elektrophoretischen Beschichtung (2 kV während 10 s) Kunststoffschichten mit folgender Dicke ausgebildet:

| | |
|---|---|
| Transparentes Halar^{®} | 80 µm |
| Blaugefärbtes Halar^{®} | 80 µm |
| Gelbgefärbtes Halar^{®} | 60 µm |
| Schwarzgefärbtes Halar^{®} | 40 µm |
| Halar^{®}-Primer | 80 µm |

Die Schichtdicke wurde gravimetrisch bestimmt, indem die abgeschiedene Masse der Kunststoffbeschichtung (vor und nach der elektrophoretischen Abscheidung) bestimmt wurde. Aus dieser Masse wurde unter Annahme einer homogenen Schichtdicke der entsprechende Wert für diese Schichtdicke berechnet.

Durch mehrfache Ausführung des geschilderten Abscheideverfahrens wurden Beschichtungen mit einer Gesamtschichtdicke von ca. 200 µm bereitgestellt. Dabei wurden sowohl dieselben Kunststoffe übereinander geschichtet als auch verschiedene Kunststoffe, d.h. beispielsweise eine Schichtabfolge aus Primer und darüber angeordneten Schichten aus transparentem oder gefärbtem ECTFE.

Sämtliche Kunststoffbeschichtungen, die in den Beispielen gemäß der Erfindung erhalten wurden, wiesen eine Durchschlagsfestigkeit von 5 kV bezogen auf die Schichtdicke von 200 µm auf. Diese Beschichtungen waren auch unter mehrfacher Anwendung eines Sterilisationsverfahrens im Autoklaven beständig, wie es in der Medizintechnik üblicherweise für die Sterilisation von bipolaren Applikatoren zur Anwendung kommt.

## Patentansprüche

1. Verfahren zum Aufbringen mindestens einer Beschichtung aus mindestens einem elektrisch isolierenden Kunststoff auf einen Applikator für Ströme, insbesondere HF-Ströme in der Chirurgie, insbesondere auf eine Klemme, auf eine Pinzette oder auf eine Schere, **dadurch gekennzeichnet, dass** die Beschichtung mindestens teilweise, vorzugsweise vollständig, durch elektrophoretische Abscheidung aus einer Suspension des Kunststoffs in mindestens einem organischen Lösungsmittel erzeugt wird, wobei die erhaltene Kunststoffbeschichtung eine elektrische Durchschlagsfestigkeit von mindestens 500 V/mm und eine Dicke zwischen 5 µm und 500 µm aufweist.

2. Verfahren nach Anspruch 1, **dadurch gekennzeichnet, dass** es sich bei dem Kunststoff um einen transparenten Kunststoff handelt.

3. Verfahren nach Anspruch 1, **dadurch gekennzeichnet, dass** es sich bei dem Kunststoff um einem gefärbten Kunststoff, vorzugsweise um einen durch den Zusatz von Farbpigmenten gefärbten Kunststoff handelt.

4. Verfahren nach einem der vorhergehenden Ansprüche, **dadurch gekennzeichnet, dass** es sich bei dem Kunststoff um ein Polyamid oder um ein Polyaryletherketon, vorzugsweise um Polyetheretherketon (PEEK) handelt.

5. Verfahren nach einem der vorhergehenden Ansprüche, **dadurch gekennzeichnet, dass** es sich bei dem Kunststoff um einen Fluorkunststoff, vorzugsweise um einen thermoplastischen Fluorkunststoff handelt, wobei insbesondere es sich bei dem Fluorkunststoff um Polytetrafluorethylen (PTFE) oder insbesondere um Polychlortrifluorethylen (PCTFE) oder Ethylenchlortrifluorethylen (ECTFE) handelt.

6. Verfahren nach einem der vorhergehenden Ansprüche, **dadurch gekennzeichnet, dass** die erhaltene Kunststoffbeschichtung eine elektrische Durchschlagsfestigkeit von mindestens 1200 V/mm, insbesondere von mindestens 2500 V/mm, aufweist, wobei vorzugsweise die elektrische Durchschlagsfestigkeit zwischen 3 kV/mm und 150 kV/mm, insbesondere zwischen 50 kV/mm und 100 kV/mm, beträgt.

7. Verfahren nach einem der vorhergehenden Ansprüche, insbesondere nach Anspruch 6, **dadurch gekennzeichnet, dass** die erhaltene Kunststoffbeschichtung eine Dicke von 30 µm bis 350 µm, insbesondere von 100 µm bis 250 µm aufweist.

8. Verfahren nach einem der vorhergehenden Ansprüche, insbesondere nach Anspruch 6, **dadurch gekennzeichnet, dass** die Kunststoffbeschichtung eine Dicke zwischen 5 µm und 100 µm, vorzugsweise von 10 µm bis 60 µm, insbesondere von 15 µm bis 30 µm aufweist.

9. Verfahren nach einem der vorhergehenden Ansprüche, **dadurch gekennzeichnet, dass** die durch elektrophoretische Abscheidung erzeugte Kunststoffbeschichtung einer Nachbehandlung durch Aufschmelzen unterzogen wird, wobei vorzugsweise die Nachbehandlung bei einer Temperatur von maximal 20 % oberhalb der Schmelztemperatur des Kunststoffs, insbesondere bei einer Temperatur von ca. 10 % oberhalb der Schmelztemperatur, erfolgt.

10. Verfahren nach Anspruch 9, **dadurch gekennzeichnet, dass** die Nachbehandlung über einen Zeitraum von weniger als 1 h, vorzugsweise von weniger als 30 min, insbesondere von weniger als 15 min, erfolgt.

11. Verfahren nach einem der vorhergehenden Ansprüche, **gekennzeichnet durch** die folgende Verfahrensabfolge:
- Ggf. Entfetten des unbeschichteten Applikators,
- ggf. Spülen mit einem Lösungsmittel und Trocknen des insbesondere entfetteten Applikators,
- ggf. Aufbringen einer Primerschicht, vorzugsweise durch elektrophoretische Abscheidung,
- ggf. Aufbringen einer farbgebenden Schicht, vorzugsweise durch elektrophoretische Abscheidung,
- Aufbringen mindestens eines elektrisch isolierenden Kunststoffs auf den ggf. vorbeschichteten Applikator durch elektrophoretische Abscheidung,
- ggf. einmaliges oder mehrmaliges Wiederholen des vorherigen Verfahrensschritts des elektrophoretischen Aufbringens der Kunststoffbeschichtung,
- ggf. Behandlung der erhaltenen Kunststoffbeschichtung mit mindestens einem Lösungsmittel,
- thermische Behandlung des mit dem Kunststoff beschichteten Applikators zur Trocknung und ggf. zur Nachbehandlung der erhaltenen Kunststoffbeschichtung durch Aufschmelzen.

12. Applikator für Ströme, insbesondere HF-Ströme in der Chirurgie, insbesondere Klemme, Pinzette oder Schere, mit mindestens einer Beschichtung aus mindestens einem elektrisch isolierenden Kunststoff, **dadurch gekennzeichnet, dass** der Kunststoff durch elektrophoretische Abscheidung aus einer Suspension des Kunststoffs in mindestens einem organischen Lösungsmittel erzeugt ist, wobei die erhaltene Kunststoffbeschichtung eine elektrische Durchschlagsfestigkeit von mindestens 500 V/mm und eine Dicke zwischen 5 µm und 500 µm aufweist, und wobei vorzugsweise es sich bei dem Kunststoff um ein Polyamid, um ein Polyaryletherketon, vorzugsweise um ein Polyetheretherketon (PEEK), oder um einen Fluorkunststoff, vorzugsweise einen thermoplastischen Fluorkunststoff, insbesondere um Polytetrafluorethylen (PTFE) oder insbesondere um Polychlortrifluorethylen (PCTFE) oder Ethylenchlortrifluorethylen (ECTFE), handelt.

13. Applikator nach Anspruch 12, **dadurch gekennzeichnet, dass** die Kunststoffbeschichtung eine elektrische Durchschlagsfestigkeit zwischen 3 kV/mm und 150 kV/mm, insbesondere zwischen 50 kV/mm und 100 kV/mm, besitzt.

## Claims

1. Method of applying at least one coating of at least one electrically insulating polymer to an applicator for currents, especially HF currents in surgery, especially to a clamp, to a pair of tweezers or to a pair of scissors, **characterized in that** the coating is produced at least partly, preferably entirely, by electrophoretic deposition from a suspension of the polymer in at least one organic solvent, where the polymer coating obtained has an electrical breakdown resistance of at least 500 V/mm and a thickness between 5 um and 500 µm.

2. Method according to Claim 1, **characterized in that** the polymer is a transparent polymer.

3. Method according to Claim 1, **characterized in that** the polymer is a coloured polymer, preferably a polymer coloured by the addition of colour pigments.

4. Method according to any of the preceding claims, **characterized in that** the polymer is a polyamide or a polyaryl ether ketone, preferably polyether ether ketone (PEEK).

5. Method according to any of the preceding claims, **characterized in that** the polymer is a fluoropolymer, preferably a thermoplastic fluoropolymer, in particular where the fluoropolymer is polytetrafluoroethylene (PTFE) or in particular polychlorotrifluoroethylene (PCTFE) or ethylene-chlorotrifluoroethylene (ECTFE).

6. Method according to any of the preceding claims, **characterized in that** the polymer coating obtained has an electrical breakdown resistance of at least 1200 V/mm, especially of at least 2500 V/mm, preferably where the electrical breakdown resistance is between 3 kV/mm and 150 kV/mm, especially between 50 kV/mm and 100 kV/mm.

7. Method according to any of the preceding claims, especially according to Claim 6, **characterized in that** the polymer coating obtained has a thickness of 30 µm to 350 µm, especially of 100 µm to 250 µm.

8. Method according to any of the preceding claims, especially according to Claim 6, **characterized in that** the polymer coating has a thickness between 5 µm and 100 µm, preferably of 10 µm to 60 µm, especially of 15 µm to 30 µm.

9. Method according to any of the preceding claims, **characterized in that** the polymer coating obtained by electrophoretic deposition is subjected to an aftertreatment by melting, preferably where the aftertreatment is effected at a temperature of at maximum 20% above the melting temperature of the polymer, especially at a temperature of about 10% above the melting temperature.

10. Method according to Claim 9, **characterized in that** the aftertreatment is effected over a period of less than 1 h, preferably of less than 30 min, especially of less than 15 min.

11. Method according to any of the preceding claims, **characterized by** the following method sequence:
- optionally degreasing the uncoated applicator,
- optionally rinsing with a solvent and drying the applicator which has especially been degreased,
- optionally applying a primer layer, preferably by electrophoretic deposition,
- optionally applying a colouring layer, preferably by electrophoretic deposition,
- applying at least one electrically insulating polymer to the optionally precoated applicator by electrophoretic deposition,
- optionally repeating the preceding method step of electrophoretic application of the polymer coating once or more than once,
- optionally treating the polymer coating obtained with at least one solvent,
- thermally treating the applicator coated with the polymer for drying and optionally for aftertreatment of the polymer coating obtained by melting.

12. Applicator for currents, especially HF currents in surgery, especially a clamp, pair of tweezers or pair of scissors, having at least one coating of at least one electrically insulating polymer, **characterized in that** the polymer has been produced by electrophoretic deposition from a suspension of the polymer in at least one organic solvent, where the polymer coating obtained has an electrical breakdown resistance of at least 500 V/mm and a thickness between 5 um and 500 um, and where the polymer is preferably a polyamide or a polyaryl ether ketone, preferably a polyether ether ketone (PEEK), or a fluoropolymer, preferably a thermoplastic fluoropolymer, in particular polytetrafluoroethylene (PTFE) or in particular polychlorotrifluoroethylene (PCTFE) or ethylene-chlorotrifluoroethylene (ECTFE).

13. Applicator according to Claim 12, **characterized in that** the polymer coating has an electrical breakdown resistance between 3 kV/mm and 150 kV/mm, especially between 50 kV/mm and 100 kV/mm.

## Revendications

1. Procédé d'application d'au moins un revêtement constitué par au moins un matériau synthétique électriquement isolant sur un applicateur de courants, en particulier des courants HF, en chirurgie, en particulier sur une pince, sur une pincette ou sur des ciseaux, **caractérisé en ce que** le revêtement est produit au moins partiellement, de préférence complètement, par dépôt électrophorétique à partir d'une suspension du matériau synthétique dans au moins un solvant organique, le revêtement en matériau synthétique obtenu présentant une rigidité diélectrique d'au moins 500 V/mm et une épaisseur entre 5 um et 500 µm.

2. Procédé selon la revendication 1, **caractérisé en ce qu'**il s'agit, pour le matériau synthétique, d'un matériau synthétique transparent.

3. Procédé selon la revendication 1, **caractérisé en ce qu'**il s'agit, pour le matériau synthétique, d'un matériau synthétique coloré, de préférence d'un matériau synthétique coloré par l'ajout de pigments colorés.

4. Procédé selon l'une des revendications précédentes, **caractérisé en ce qu'**il s'agit, pour le matériau synthétique, d'un polyamide ou d'une polyaryléthercétone, de préférence de polyétheréthercétone (PEEK).

5. Procédé selon l'une des revendications précédentes, **caractérisé en ce qu'**il s'agit, pour le matériau synthétique, d'un matériau synthétique fluoré, de préférence d'un matériau synthétique fluoré thermoplastique, le matériau synthétique fluoré étant en particulier le polytétrafluoroéthylène (PTFE) ou en particulier le polychlorotrifluoroéthylène (PCTFE) ou l'éthylènechlorotrifluoroéthylène (ECTFE).

6. Procédé selon l'une des revendications précédentes, **caractérisé en ce que** le revêtement en matériau synthétique obtenu présente une rigidité diélectrique d'au moins 1200 V/mm, en particulier d'au moins 2500 V/mm, la rigidité diélectrique étant de préférence située entre 3 kV/mm et 150 kV/mm, en particulier entre 50 kV/mm et 100 kV/mm.

7. Procédé selon l'une des revendications précédentes, en particulier selon la revendication 6, **caractérisé en ce que** le revêtement en matériau synthétique obtenu présente une épaisseur de 30 um à 350 um, en particulier de 100 um à 250 µm.

8. Procédé selon l'une des revendications précédentes, en particulier selon la revendication 6, **caractérisé en ce que** le revêtement en matériau synthétique présente une épaisseur entre 5 um et 100 um, de préférence de 10 um à 60 um, en particulier de 15 um à 30 um.

9. Procédé selon l'une des revendications précédentes, **caractérisé en ce que** le revêtement en matériau synthétique produit par dépôt électrophorétique est soumis à un post-traitement par fusion, le post-traitement étant de préférence effectué à une température supérieure d'au maximum 20% à la température de fusion du matériau synthétique, en particulier à une température supérieure d'environ 10% à la température de fusion.

10. Procédé selon la revendication 9, **caractérisé en ce que** le post-traitement est effectué sur une durée de moins de 1 h, de préférence de moins de 30 min, en particulier de moins de 15 min.

11. Procédé selon l'une des revendications précédentes, **caractérisé par** le déroulement de procédé suivant :
- le cas échéant dégraissage de l'applicateur non revêtu,
- le cas échéant rinçage par un solvant et séchage de l'applicateur en particulier dégraissé,
- le cas échéant application d'une couche d'apprêt, de préférence par dépôt électrophorétique,
- le cas échéant application d'une couche conférent une couleur, de préférence par dépôt électrophorétique,
- application d'au moins un matériau synthétique électriquement isolant sur l'applicateur le cas échéant prérevêtu par dépôt électrophorétique,
- le cas échéant répétition unique ou multiple de l'étape de procédé précédente d'application électrophorétique du revêtement en matériau synthétique,
- le cas échéant traitement du revêtement en matériau synthétique obtenu par au moins un solvant,
- traitement thermique de l'applicateur revêtu par le matériau synthétique en vue du séchage et le cas échéant du post-traitement du revêtement en matériau synthétique obtenu par fusion.

12. Applicateur de courants, en particulier de courants HF en chirurgie, en particulier une pince, une pincette ou des ciseaux, présentant au moins un revêtement en au moins un matériau synthétique électriquement isolant, **caractérisé en ce que** le matériau synthétique est produit par dépôt électrophorétique à partir d'une suspension du matériau synthétique dans au moins un solvant organique, le revêtement en matériau synthétique obtenu présentant une rigidité diélectrique d'au moins 500 V/mm et une épaisseur entre 5 um et 500 um, et le matériau synthétique étant de préférence un polyamide, une polyaryléthercétone, de préférence une polyétheréthercétone (PEEK), ou un matériau synthétique fluoré, de préférence un matériau synthétique fluoré thermoplastique, en particulier le polytétrafluoroéthylène (PTFE) ou en particulier le polychlorotrifluoroéthylène (PCTFE) ou l'éthylènechlorotrifluoroéthylène (ECTFE).

13. Applicateur selon la revendication 12, **caractérisé en ce que** le revêtement matériau synthétique possède une rigidité diélectrique située entre 3 kV/mm et 150 kV/mm, en particulier entre 50 kV/mm et 100 kV/mm.
